# EUROPEAN PATENT APPLICATION

(11) **EP 3 492 607 A2**
(43) Date of publication of application: **05.06.2019**
(21) Application number: 18209223.9
(22) Date of filing: 29.11.2018
(51) Int. Cl.: C12Q 1/6886

(54) **URINARY MIRNAS FOR THE IN VITRO DIAGNOSIS OF BLADDER CANCER**

(30) Priority: 30.11.2017 IT 201700138247
(71) Applicant: Università Degli Studi Di Torino, 10123 Torino (IT); Italian Institute for Genomic Medicine (IIGM), 10126 Torino (IT)
(72) Inventor: MATULLO, Giuseppe, I-10024 MONCALIERI (Torino) (IT); PARDINI, Barbara, I-10126 TORINO (IT)
(74) Representative: Comoglio, Elena

(57) **Abstract**

The object of the present invention is a method for analysing a biological sample from a subject for the diagnosis and sub-classification of bladder cancer, wherein the method comprises the following steps:
a) collecting the miRNAs contained in a biological fluid of a subject;
b) determining the expression profile of a predetermined series of miRNAs;
c) comparing said expression profile with the expression profile of a reference sample, to determine the different expression in the sample compared to a reference sample.

## Description

The present invention relates to a method for analysing a biological sample from a subject for the diagnosis and sub-classification of bladder cancer, wherein the method comprises the following steps:
a) collecting the miRNAs contained in a biological fluid of a subject;
b) determining the expression profile of a predetermined series of miRNAs;
c) comparing said expression profile with the expression profile of a reference sample, to determine the different expression in the sample compared to a reference sample.

### State of the art

Bladder cancer (BC) is among the most frequent diseases worldwide, with about 429,000 new cases in 2012 [1]. BC is a highly heterogeneous disease. The majority of cases (70%) are non-muscle-invasive BCs (NMIBCs), which are restricted to the mucosa or the submucosa, with superficial and non-infiltrating lesions. The remaining subset of cases are classified as muscle-invasive BCs (MIBCs) [2]. A fairly high percentage of NMIBCs (50-70%) will recur in the same subject and approximately 10-30% will turn into MIBCs [3].

BC screening and early diagnosis play a primary role in improving patients' survival and quality of life. Urinary cytology is currently the most commonly used non-invasive analysis for BC detection, but has limited value due to its poor sensitivity, especially for low-grade lesions [4]. Cystoscopy-guided biopsy for histological evaluation can offer high diagnostic accuracy, but is invasive and inconvenient for patients.

BC represents a significant economic and social challenge, as the high recurrence rate requires continuous cystoscopic surveillance [5].

MicroRNAs (miRNAs) are a class of non-coding RNAs, which act as post-transcriptional regulators in gene expression silencing by binding to complementary messenger RNA (mRNA). Deregulated miRNA profiles have been associated with numerous tumours, including BC [6]. Free or vesicle-bound circulating miRNAs are abundantly present in a variety of biofluids, including plasma, saliva and urine [7, 8].

There is a strongly felt need for non-invasive analyses and more sensitive molecular biomarkers to improve current strategies for the detection and monitoring of this cancer.

### Description of the invention

The object of the present invention is a non-invasive method for the diagnosis of bladder cancer and the sub-classification thereof (muscle-invasive and non-muscle-invasive; Grade 1-2 or 3).

The authors of the present invention surprisingly demonstrated a strong and specific correlation between the miRNA profile and bladder cancer subclasses.

First of all, an miRNA profile for early diagnosis of bladder cancer is described herein.

An "miRNA" is a small, naturally occurring non-coding RNA from about 17 to about 25 nucleotides (nt) in length in its biologically active form, which negatively regulates mRNA translation in a sequence-specific manner. The identified miRNAs are stored in the miRBase miRNA database (http://microma.sanger.ac.uk/).

A "sample", as defined herein, is a small part of a subject representing the whole, which may consist of a body fluid sample. Body fluid samples may be blood, plasma, serum, urine, sputum, cerebrospinal fluid, milk, or ductal fluid samples, which may be fresh, frozen or fixed. Samples can be removed surgically, by extraction, for example, with hypodermic needles or other types of needles, microdissection or laser capture. The sample should contain any biological material suitable for detecting the desired biomarker (miRNA). In a preferred embodiment, for the purposes of the present invention, said "sample" is urine.

A "reference sample", as used herein, means a sample obtained from individuals, preferably two or more individuals, known to be not suffering from bladder cancer. Suitable reference expression levels of a given miRNA are determined by measuring the expression levels of said miRNA in several control subjects, wherein control subjects are intended to mean, for example, subjects belonging to the same ethnic group as the tested subject. In a preferred embodiment, the reference sample is obtained from a pool of healthy subjects. The miRNA expression profile in the reference sample can be preferably generated from a population of two or more subjects; for example, the population may include 3, 4, 5, 10, 15, 20, 30, 40, 50 or more subjects.

A "subject", as used herein, refers to a human being.

In the present context, the expression "diagnosis" or "diagnose" refers to how the person skilled in the art can evaluate and also determine whether an individual is affected by a particular disease or condition.

Along with the diagnosis, the clinical prognosis of the disease is also an area of great concern and interest. It is important to know the stage and progression rate of the disease in order to plan the most effective therapy. If a more accurate prognosis can be made, then a more appropriate and, in some cases, less burdensome therapy for the patient can be adopted.

Moreover, the expression "method of diagnosis", as used herein, refers to a method that may essentially consist of these steps, or may include additional steps. However, it should be understood that the method, in a preferred embodiment, is a method carried out *in vitro,* i.e., is not carried out in the human or animal body.

In a first embodiment, an *in vitro* method for the diagnosis of bladder cancer is claimed herein, the method comprising the following steps:
a) determining the expression levels of a series of miRNAs comprising at least miR-30a-5p (SEQ ID no. 3), miR-486-5p (SEQ ID no. 5), let-7c-5p (SEQ ID no. 4) in a sample of biological fluid from a subject;
b) comparing said expression levels with the expression levels of the same miRNAs in a reference sample, wherein a difference is indicative of bladder cancer.

In a further embodiment, a method is described for the sub-classification of bladder cancer, wherein said cancer is a Grade 1, 2 or 3 muscle-invasive or non-muscle-invasive cancer.

Object of the present invention is an expression level profile of a series of miRNAs characterizing each bladder cancer subclass. In particular, a sample from a subject suffering from muscle-invasive bladder cancer is assessed, with respect to a reference sample, for altered expression levels at least of the following miRNAs: miR-30a-5p (SEQ ID no. 3), let-7c-5p (SEQ ID no. 4), miR-486-5p (SEQ ID no. 5), miR-205-5p (SEQ ID no. 6), miR-451 (SEQ ID no. 7), miR-25-3p (SEQ ID no. 8), miR-7-1-5p (SEQ ID no. 9) miR-21-5p (SEQ ID no. 1), miR-106b-3p (SEQ ID no. 2), miR-146a-5p (SEQ ID no. 10).

A sample from a subject suffering from Grade 1-2 non-muscle-invasive bladder cancer is assessed, with respect to a reference sample, for altered expression levels at least of the following miRNAs: miR-30a-5p (SEQ ID no. 3), let-7c-5p (SEQ ID no. 4), miR-486-5p (SEQ ID no. 5), miR-205-5p (SEQ ID no. 6), miR-30c-2-5p (SEQ ID no. 11) miR-151a-3p (SEQ ID no. 12), let-7i-5p (SEQ ID no. 13).

A sample from a subject suffering from Grade 3 non-muscle-invasive bladder cancer is assessed for altered expression levels at least of the following miRNAs: miR-30a-5p (SEQ ID no. 3), let-7c-5p (SEQ ID no. 4), miR-486-5p (SEQ ID no. 5), miR-21-5p (SEQ ID no. 1), miR-106b-3p (SEQ ID no. 2), miR-30c-2-5p (SEQ ID no. 11), miR-151a-3p (SEQ ID no. 12), miR-200c-3p (SEQ ID no. 19), miR-183-5p (SEQ ID no. 17), miR-185-5p (SEQ ID no. 18), miR-10b-5p (SEQ ID no. 15), miR-224-5p (SEQ ID no. 20), miR-98-5p (SEQ ID no. 22) and miR-148b-3p (SEQ ID no. 16).

Said miRNAs are collected from a biological fluid of a subject and the expression level of each of them is determined according to methods known to those skilled in the art.

The method according to the present invention considerably lowers the costs related to bladder cancer diagnosis and therapeutic monitoring, avoiding the need for cystoscopy, with parallel improvement in patients' quality of life.

Compared to non-invasive systems already available, the new system has a greater sensitivity in distinguishing between subgroups, thus allowing the identification of subjects with grade G3 non-muscle-invasive cancer, the subcategory with the highest risk of progression and recurrence.

### Description of the figures

Figure 1: Heatmap of the miRNAs selected in the Discovery step differentially expressed in A) NMIBC G1 + G2 and controls; B) NMIBC G3 and controls, and C) MIBC and controls.
Figure 2: Graphs of expression levels of selected miRNAs with a significant trend (adjusted p-value <0.05) in healthy controls and MIBC patients.
Figure 3: Area under the ROC curve (AUC) for model 1 (comprising age and smoking habit as a risk category, in grey) and model 2 (which includes model 1 plus expression levels of miR-30a-5p (SEQ ID no. 3), let-7c-5p (SEQ ID no. 4) and miR-486-5p (SEQ ID no. 5); in black).

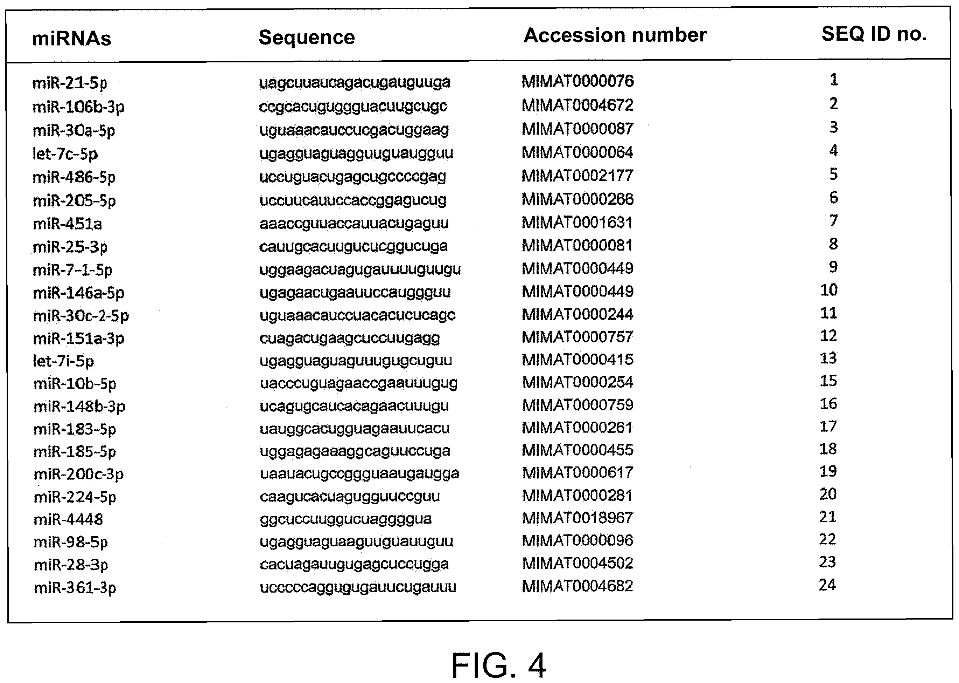

Figure 4: Summary scheme of the miRNAs used, with accession number and SEQ ID no.

### Detailed description of the invention:

### Materials and Methods

### Case Histories

The population included in the study is composed of men enrolled in the Turin Bladder Cancer Study (designated as TBCS) [1,2]. All subjects gave written consent to participate in the study, according to the Helsinki declaration.

Blood and urine samples were collected from the subjects. The patients were all newly diagnosed cases histologically confirmed as bladder cancer (BC). Controls were enrolled among men treated for non-neoplastic diseases (prostatic hyperplasia, cystitis and others) or among other patients treated for benign diseases, such as hernias, vasculopathy, asthma, infarction, etc. Patients with cancer, kidney or liver disease, or other smoking-related diseases were excluded from the study.

The cases were interviewed to obtain demographic information on possible cancer risk factors (for example, the detailed history of cancer cases in the family). BC patients were monitored by urologists with periodic cystoscopic examinations. Clinical information, including the types of therapy administered (for example *Bacillus Calmette-Guerin* (BCG), chemotherapy and radiotherapy), was provided by the urologists who monitored the patients. Details on the cases included in the study are shown in Table 1.

**Table 1:**

| | | **Discovery (n)** | | **Validation (n)** | | **Overall (n)** | |
|---|---|---|---|---|---|---|---|
| | | Cases (66) | Controls (49) | Cases (46) | Controls (16) | Cases (112) | Controls (65) |
| Age | Mean (median) | 64.27 (65.02) | 64.64 (65.60) | 64.63 (66.83) | 65.14 (70.25) | 64.42 (65.89) | 64.76 (66.53) |
| | Ranges | 44.92-74.10 | 46.44-74.91 | 46.78-74.64 | 41.92-74.49 | 44.92-74.64 | 41.92-74.91 |
| Smoking | Non-smokers | 7 | 5 | 3 | 6 | 10 | 11 |
| | Former smokers | 34 | 26 | 22 | 6 | 56 | 32 |
| | Current smokers | 25 | 18 | 21 | 3 | 46 | 21 |
| | n.a. | 0 | 0 | 0 | 1 | 0 | 1 |
| # Grade | 1 | 14 | | 16 | | 30 | |
| | 2 | 25 | | 27 | | 52 | |
| | 3 | 27 | | 3 | | 30 | |
| # G | High grade | 41 | | 14 | | 55 | |
| | Not high grade | 25 | | 32 | | 57 | |
| Tumour stage | is | 3 | | 1 | | 4 | |
| | a | 29 | | 35 | | 64 | |
| | 1 | 24 | | 8 | | 32 | |
| | >2 | 10 | | 0 | | 10 | |
| | X | 0 | | 2 | | 2 | |
| Tumour type* | NMIBC | 56 | | 46 | | 102 | |
| | MIBC | 10 | | 0 | | 10 | |
| Risk^{#}* | 1 | 11 | | 15 | | 26 | |
| | 2 | 18 | | 22 | | 40 | |
| | 3 | 26 | | 9 | | 35 | |
| | MIBC | 10 | | 0 | | 10 | |
| | n.a. | 1 | | 0 | | 1 | |
| Recurrences | yes | 25 | | 15 | | 40 | |
| | no | 41 | | 31 | | 72 | |
| Progression | yes | 2 | | 0 | | 2 | |
| | no | 64 | | 46 | | 110 | |
| Status | alive | 58 | | 44 | | 102 | |
| | dead | 8 | | 2 | | 10 | |

| | | | | | | | |
|---|---|---|---|---|---|---|---|
| * EAU Guidelines on Non-Muscle-invasive Urothelial Carcinoma of the Bladder: Update 2013; European urology 64(2013): 639-653. # Cheng L. Cancer: 2000; 88(7):1513-6. and Montironi R. Lopez-Beltran A. International Journal of Surgical Pathology. 13(2): 143-53, 2005 Apr | | | | | | | |

### RNA extraction and small non-coding RNA sequencing (smallRNA-seq)

Total RNA was extracted from urine supernatant samples by using the urine miRNA purification kit (Urine microRNA purification kit, Norgen Biotek, Canada) according to the standard protocol.

Small RNA transcripts were converted into cDNA libraries provided with a unique identifier for each sample with the NEBNext Multiplex Small RNA Library Prep Set for Illumina (New England BioLabs, USA). The libraries were sequenced on Illumina HiSeq2000 (Illumina, USA).

### miRNA quantification by quantitative PCR (qPCR)

miRNAs candidates for the role of biomarkers were validated in urine samples by using the miRCURY LNA™ Universal microRNA RT PCR system (Exiqon, Denmark). Reverse transcription (RT) was performed by using the Universal cDNA synthesis kit II (Exiqon, Denmark) according to the manufacturer's instructions with the addition of a spike-in (UniSp6) to the RT reaction.

### Computational and statistical analyses

Raw reads quality-check, adapter clipping and mapping were performed as in [4]. After the mapping of the reads, a matrix of integer values designated as counting matrix was created. The value in the i-th row and the j-th column of the matrix show how many reads were unequivocally assigned to mature miRNA i in sample j. The unwanted contribution to the variability present in the samples (e.g. batch effect) was estimated by using the functions implemented in the SVA package.

The miRNA expression levels were measured in the following bladder cancer subtypes: invasive cancer - muscle-invasive Bladder Cancer (MIBC), non-invasive-cancer - non muscle-invasive Bladder Cancer (NMIBC), which, in turn, is divided into G1+G2 and G3, versus controls. Candidate miRNAs were selected by using the following approaches (adapted from [5]):
1) identification of differently expressed miRNAs (DEmiRNAs) by using negative binomial generalized linear models implemented in the DESeq2 package in Bioconductor [9];
2) computation of a regression model in which the individual levels of the variables, i.e. the individual miRNA expression levels, are used to predict the class label (i.e. BC patients or healthy controls) of each subject [10] (Predictive Power (PP) calculation);
3) selection of the resulting relevant miRNAs from the literature. In order to select the most relevant miRNAs for the validation, the analysis was restricted to the significant DEmiRNAs (adjusted FDR (False Discovery Rate) p < 0.05) associated with a mean read count at least higher than 300 (defined as abundant DEmiRNAs) and to miRNAs associated with a PP ≥ 0.70. Because of the still high number of candidate miRNAs for replica/validation by qPCR, among all miRNAs forming part of a cluster only the most representative one was selected, identified by the normalized count values (= the abundance) in the tumour classes.

Endogenous controls for the normalization of the qPCR were identified by adapting the pipeline developed by Eisenberg and Levanon [6]. In short, the miRNAs identified with the NGS data were selected by taking into account the individual count and on the basis of the following criteria: 1) at least 20 reads for each sample; 2) a log2 standard deviation value <14.50; 3) a log2 fold change ranging from -4 to 7.

The Delta Ct (DCt) values were obtained by normalizing the data from the candidate miRNAs to endogenous controls identified by NGS.

Differential miRNA expression levels (expressed as fold-change and calculated as log2^{-DDCt)} between BC subtypes and controls were assessed by logistic regression adjusted for age and smoking habit. Results with p-values < 0.05 were considered statistically significant.

Target genes for miRNAs of interest were retrieved by miRWalk2.0 database [7]. EnrichR was used for gene ontological analysis and for gene pathway enrichment analyses [8, 11]. The open-access dataset of MIBC patient cases from The Cancer Genome Atlas (TCGA) project was used for further comparative analysis.

### Example 1: Discovery Phase

In total, 114 samples (66 BC cases and 48 controls) were included in the discovery phase and analysed by smallRNA-seq. Among the cases, 10 were diagnosed as MIBCs, while 56 were NMIBCs (39 G1+G2 and 17 G3).

Analysis of the raw reads has led to the definition of the starting count matrix composed of 114 samples and 1822 miRNAs having at least one read in one sample. However, in subsequent analyses of the Discovery phase, only miRNAs with at least 20 reads considering all samples (1787 final miRNAs) were included.

In the comparison between NMIBC G1 + G2 and controls, 98 DEmiRNAs were identified, 14 of which had high abundance of reads. Five miRNAs were associated with a PP higher than 0.7 by logistic regression analysis, among which miR-30a-5p (SEQ ID no. 3), miR-205-5p (SEQ ID no. 6), let-7c (SEQ ID no. 4), also included in the list of DEmiRNAs. Other miRNAs were identified among those with the most abundant read counts in order to eventually define seven miRNA candidates as biomarkers of the NMIBC G1 + G2 bladder cancer type, listed in the second block of Table 2, which shows the list of miRNA candidates as biomarkers for the three tumour subclasses.

**Table 2**

| | **Mean read counts** | **log2 Fold Change** | **adj *P* (FDR)** | **PP** |
|---|---|---|---|---|
| **Candidate miRNAs for MIBC** | | | | |
| miR-21-5p (SEQ ID no. 1) | 29223 | 1.42 | 0.01 | 0.85 |
| miR-106b-3p (SEQ ID no. 2) | 130 | 2.53 | 0.00 | 0.85 |
| miR-30a-5p (SEQ ID no. 3) | 19453 | -1.80 | 0.05 | 0.85 |
| let-7c-5p (SEQ ID no. 4) | 1497 | -1.62 | 0.02 | 0.85 |
| miR-486-5p (SEQ ID no. 5) | 819 | 3.68 | 0.01 | <0.70 |
| miR-205-5p (SEQ ID no. 6) | 376 | 2.97 | 0.00 | 0.9 |
| miR-451a (SEQ ID no. 7) | 1004 | 3.40 | 0.04 | <0.70 |
| miR-25-3p (SEQ ID no. 8) | 588 | 1.99 | 0.02 | <0.70 |
| miR-7-1-5p (SEQ ID no. 9) | 518 | 3.32 | 0.05 | 0.85 |
| miR-146a-5p (SEQ ID no. 10) | 420 | 2.49 | 0.04 | <0.70 |

| **Candidate miRNAs for NMIBC G1+G2** | | | | |
|---|---|---|---|---|
| miR-30c-2-5p (SEQ ID no. 11) | 2106 | -0.73 | 0.00 | <0.70 |
| miR-151a-3p (SEQ ID no. 12) | 2873 | 0.34 | 0.02 | <0.70 |
| miR-30a-5p (SEQ ID no. 3) | 27229 | -0.63 | 0.00 | 0.73 |
| let-7c-5p (SEQ ID no. 4) | 2644 | -0.43 | 0.04 | 0.71 |
| miR-486-5p (SEQ ID no. 5) | 1868 | 1.94 | 0.02 | <0.70 |
| miR-205-5p (SEQ ID no. 6) | 479 | 1.82 | 0.00 | 0.73 |
| let-7i-5p (SEQ ID no. 13) | 5038 | 0.55 | 0.03 | <0.70 |

| **Candidate miRNAs for NMIBC G3** | | | | |
|---|---|---|---|---|
| miR-21-5p (SEQ ID no. 1) | 38624 | 0.87 | 0.03 | 0.76 |
| miR-106b-3p (SEQ ID no. 2) | 184 | 2.30 | 0.00 | 0.88 |
| miR-30a-5p (SEQ ID no. 3) | 29232 | -1.67 | 0.00 | 0.76 |
| let-7c-5p (SEQ ID no. 4) | 2939 | -1.17 | 0.03 | <0.70 |
| miR-486-5p (SEQ ID no. 5) | 2918 | 3.14 | 0.00 | <0.70 |
| miR-30c-2-5p (SEQ ID no. 11) | 2241 | -1.47 | 0.01 | <0.70 |
| miR-151a-3p (SEQ ID no. 12) | 3884 | 0.51 | 0.03 | <0.70 |
| miR-10b-5p (SEQ ID no. 15) | 28018 | -1.88 | 0.00 | 0.85 |
| miR-148b-3p (SEQ ID no. 16) | 660 | 0.95 | 0.00 | 0.85 |
| miR-183-5p (SEQ ID no. 17) | 1414 | 1.50 | 0.00 | <0.70 |
| miR-185-5p (SEQ ID no. 18) | 381 | 2.10 | 0.00 | 0.76 |
| miR-200c-3p (SEQ ID no. 19) | 9145 | 1.11 | 0.00 | 0.76 |
| miR-224-5p (SEQ ID no. 20) | 488 | 2.97 | 0.00 | 0.79 |
| miR-4448 (SEQ ID no. 21) | 7834 | -2.27 | 0.01 | <0.70 |
| miR-98-5p (SEQ ID no. 22) | 459 | 1.01 | 0.01 | 0.79 |

| | | | | |
|---|---|---|---|---|
| Abbreviations: FDR, false discovery rate, PP predictive power, MIBC muscle-invasive bladder cancer, NMIBC non-muscle-invasive bladder cancer | | | | |

In the second comparison (NMIBC G3 vs. controls), 263 miRNAs were found to be differently expressed between cases and controls (reference samples). The logistic regression analysis identified 61 miRNAs associated with a PP higher than 0.70.

For the validation/replica phase, among these 3 miRNAs were selected that had a PP>0.85 and a high number of read counts. The 15 definitive DEmiRNAs, selected as described above, are: miR-30a-5p (SEQ ID no. 3), let-7c (SEQ ID no. 4), miR-486-5p (SEQ ID no. 5), miR-183-5p (SEQ ID no. 17), miR-185-5p (SEQ ID no. 18), miR-106b-3p (SEQ ID no. 2), miR-98-5p (SEQ ID no. 22), miR-4448 (SEQ ID no. 21), miR-30c-2-5p (SEQ ID no. 11), miR-151a-3p (SEQ ID no. 12), miR-200c-3p (SEQ ID no. 19), miR-21-5p (SEQ ID no. 1), miR-10b-5p (SEQ ID no. 15), miR-224-5p (SEQ ID no. 20), and mir-148b-3p (SEQ ID no. 16), as shown in the third block of Table 2.

Finally, MIBC cases were compared to controls. Out of the 33 significant DEmiRNAs, 11 had a mean read value > 300. In addition, 44 miRNAs (60% of which also identified as DEmiRNAs) had a PP higher than 0.80. Ten miRNAs were selected as candidate biomarkers to distinguish MIBC cases taking into account both criteria, shown in the first block of Table 2.

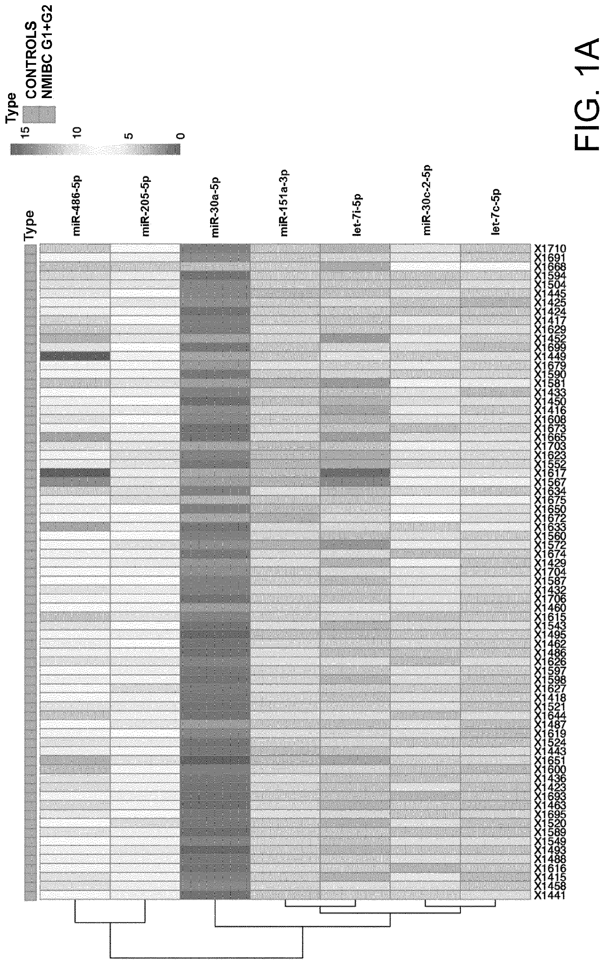

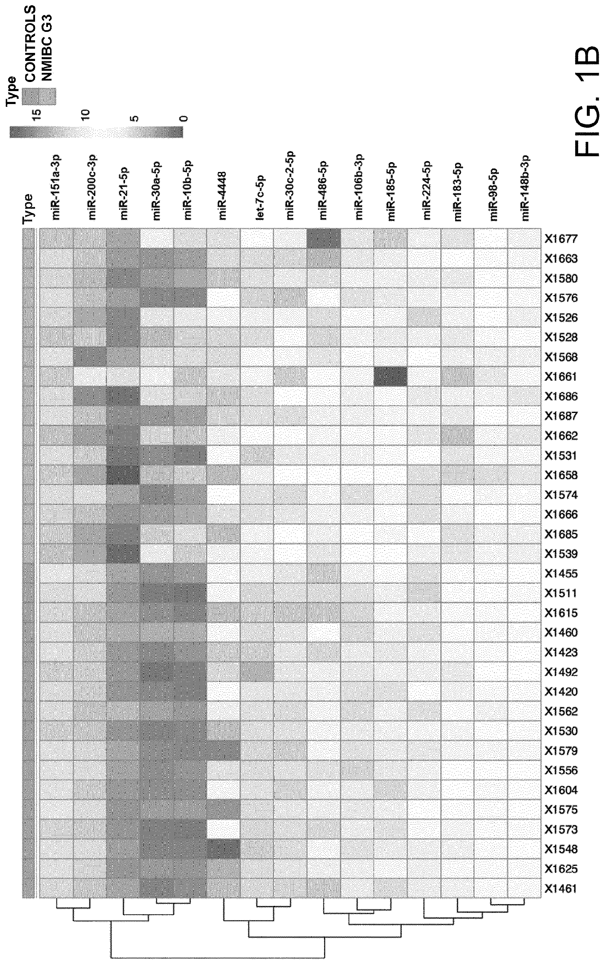

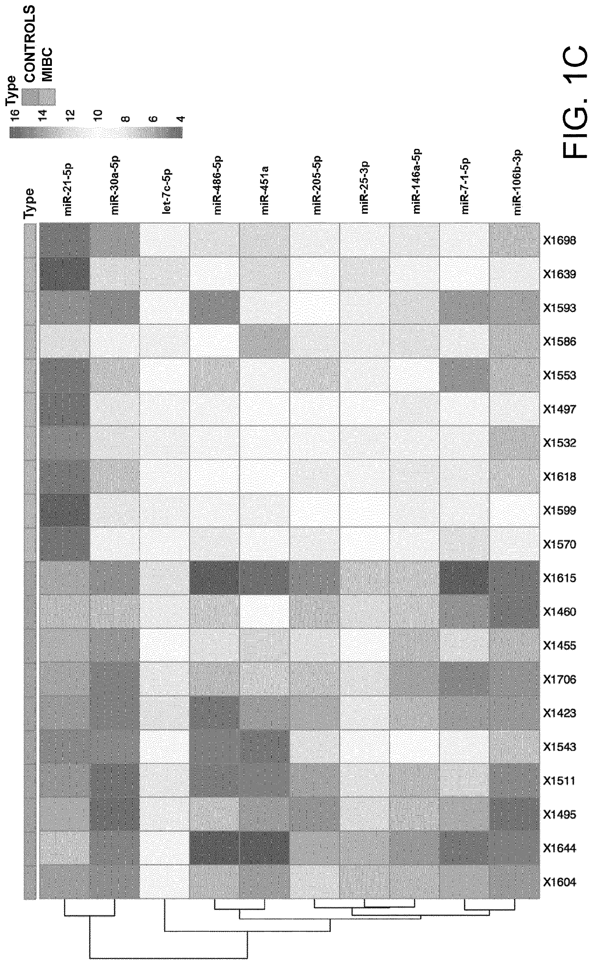

In total, eight miRNAs among those selected for NMIBC G1+G2 (n=7), NMIBC G3 (n=15) and MIBC (n=10) were in common among the different bladder cancer subtypes, with three of them in common among all three groups. Finally, 21 miRNAs were selected for the Validation/Replica phase. The heatmaps for all miRNAs resulting from all comparisons are shown in Figure 1.

To select appropriate endogenous controls for qPCR data normalization, the NGS data were analysed by using an adaptation of the pipeline developed by Eisenberg and Levanon [6]. Two reference genes, hsa-miR-28-3p (SEQ ID no. 23) and hsa-miR-361-3p (SEQ ID no. 24) met the selection criteria and were used as endogenous controls in the qPCR analyses.

### Example 2: Validation/Replica

Twenty-one candidate miRNAs from the Discovery phase were validated by qPCR on the same set of BC cases and controls employed in the NGS small RNA-seq analyses (validation set) and in urine samples from other 46 BC cases (43 G1+G2 and 3 G3) and 16 controls (replica set). miR-28-3p (SEQ ID no. 23) and miR-361-3p (SEQ ID no. 24) were included in the validation/replica as endogenous controls for normalization. miR-4448 (SEQ ID no. 21) expression levels could not be measured by qPCR.

The validation/replica results are shown in Table 3.

**Table 3**

| | | **Validation** | | | **Validation + Replica** | | |
|---|---|---|---|---|---|---|---|
| | **miRNA** | **Log2 Fold Change** | **P** | **adj P** | **Log2 Fold Change** | **P** | **adj P** |
| MIBC | miR-21-5p (SEQ ID no. 1) | 0.65 | 0.309 | 0.386 | 0.73 | 0.271 | 0.338 |
| | miR-106b-3p (SEQ ID no. 2) | 1.33 | 0.069 | 0.116 | 1.58 | 0.054 | 0.107 |
| | **miR-30a-5p (SEQ ID no. 3)** | -2.43 | 0.002 | 0.011 | -2.12 | 0.006 | 0.017 |
| | **let-7c-5p (SEQ ID no. 4)** | -1.29 | 0.144 | 0.205 | -1.04 | 0.234 | 0.312 |
| | **miR-486-5p (SEQ ID no. 5)** | 2.55 | 0.026 | 0.058 | 2.75 | 0.017 | 0.038 |
| | miR-205-5p (SEQ ID no. 6) | 1.84 | 0.005 | 0.017 | 1.92 | 0.012 | 0.029 |
| | miR-451a (SEQ ID no. 7) | 3.13 | 0.011 | 0.031 | 3.57 | 0.004 | 0.014 |
| | miR-25-3p (SEQ ID no. 8) | 1.97 | 0.005 | 0.017 | 2.21 | 0.004 | 0.014 |
| | miR-7-1-5p (SEQ ID no. 9) | 2.49 | 0.002 | 0.011 | 2.74 | 0.001 | 0.012 |
| | miR-146a-5p (SEQ ID no. 10) | 1.00 | 0.109 | 0.168 | 1.14 | 0.131 | 0.193 |
| NMIBC G1+G2 | **miR-30a-5p (SEQ ID no. 3)** | -0.52 | 0.304 | 0.656 | -0.28 | 0.482 | 0.508 |
| | **let-7c-5p (SEQ ID no. 4)** | -0.18 | 0.764 | 0.858 | 0.25 | 0.553 | 0.553 |
| | **miR-486-5p (SEQ ID no. 5)** | 1.67 | 0.059 | 0.197 | 1.63 | 0.017 | 0.073 |
| | miR-30c-2-5p (SEQ ID no. 11) | -1.08 | 0.022 | 0.144 | -0.58 | 0.149 | 0.248 |
| | miR-151a-3p (SEQ ID no. 12) | -0.26 | 0.494 | 0.657 | 0.37 | 0.265 | 0.353 |
| | miR-205-5p (SEQ ID no. 6) | 1.76 | 0.000 | 0.007 | 1.60 | 0.000 | 0.002 |
| | let-7i-5p (SEQ ID no. 13) | 0.31 | 0.427 | 0.656 | 0.76 | 0.026 | 0.076 |
| NMIBC G3 | miR-21-5p (SEQ ID no. 1) | 1.36 | 0.005 | 0.008 | 1.29 | 0.007 | 0.011 |
| | miR-106b-3p (SEQ ID no. 2) | 1.67 | 0.001 | 0.002 | 1.94 | 0.000 | 0.001 |
| | **miR-30a-5p (SEQ ID no. 3)** | -0.97 | 0.127 | 0.149 | -0.78 | 0.178 | 0.210 |
| | **let-7c-5p (SEQ ID no. 4)** | 1.25 | 0.088 | 0.110 | 1.13 | 0.097 | 0.121 |
| | **miR-486-5p (SEQ ID no. 5)** | 3.13 | 0.001 | 0.002 | 3.37 | 0.000 | 0.001 |
| | miR-30c-2-5p (SEQ ID no. 11) | -1.56 | 0.001 | 0.002 | -1.19 | 0.019 | 0.027 |
| | miR-151a-3p (SEQ ID no. 12) | 1.22 | 0.001 | 0.002 | 1.41 | 0.001 | 0.002 |
| | miR-200c-3p (SEQ ID no. 19) | 1.53 | 0.000 | 0.001 | 1.63 | 0.000 | 0.001 |
| | miR-4448 (SEQ ID no. 21) | na | | | na | na | na |
| | miR-183-5p (SEQ ID no. 17) | 1.96 | 0.000 | 0.000 | 1.98 | 0.000 | 0.000 |
| | miR-185-5p (SEQ ID no. 18) | 0.86 | 0.015 | 0.021 | 0.87 | 0.022 | 0.029 |
| | miR-98-5p (SEQ ID no. 22) | 0.34 | 0.473 | 0.526 | 0.00 | 0.995 | 0.995 |
| | miR-148b-3p (SEQ ID no. 16) | 0.09 | 0.887 | 0.887 | -0.03 | 0.951 | 0.995 |
| | miR-10b-5p (SEQ ID no. 15) | -1.69 | 0.018 | 0.024 | -1.64 | 0.005 | 0.008 |
| | miR-224-5p (SEQ ID no. 20) | 2.76 | 0.000 | 0.000 | 2.97 | 0.000 | 0.000 |

The normalized expression levels obtained by qPCR showed levels comparable to those obtained in NGS for all miRNAs, although with different significance. A significant reduction in the expression levels of miR-30c-2-5p (SEQ ID no. 11) (p = 0.02) and an increase in the levels of miR-205-5p (SEQ ID no. 6) were observed in patients suffering from NMIBC G1+G2 tumour compared to controls (validation group, Table 3). In all NMIBC G1+G2 cases (Validation+Replica) compared to all controls, miR-205-5p (SEQ ID no. 6), miR-486-5p (SEQ ID no. 5) and let-7i-5p (SEQ ID no. 13) were found to be significantly over-expressed (p = 0.0001; p = 0.02 and p = 0.03, respectively) as in the Discovery phase.

In NMIBC G3 patients and controls, 10 out of the 15 miRNAs tested by qPCR were found to be significantly differentially expressed both in Validation and in Validation+Replica. In particular, miR-21-5p (SEQ ID no. 1), miR-106b-3p (SEQ ID no. 2), miR-486-5p (SEQ ID no. 5), miR-151a-3p (SEQ ID no. 12), miR-200c-3p (SEQ ID no. 19), miR-183-5p (SEQ ID no. 17), miR-185-5p (SEQ ID no. 18) and miR-224- 5p (SEQ ID no. 20) (in Validation+Replica p-values ranging from 1.94×10-6 to 0.02) were found to be over-expressed in NMIBC G3 cases, while miR-30c-2-5p (SEQ ID no. 11) and miR-10b-5p (SEQ ID no. 15) were found to be down-regulated in the same cases (p = 0.02 and p = 0.005, respectively; Table 3).

In MIBC cases, miR-486-5p (SEQ ID no. 5), miR-205-5p (SEQ ID no. 6), miR-451a (SEQ ID no. 7), miR-25-3p (SEQ ID no. 8) and miR-7-1-5p (SEQ ID no. 9) were found to have increased expression levels compared to controls (p-value comprised between 0.001 and 0.02), while miR-30a-5p (SEQ ID no. 3) exhibited significantly lower (p = 0.006) expression levels (Validation+Replica, Table 3).

The results from the smallRNA-seq analysis for MIBC cases were further compared with those contained in the TCGA dataset [12]. Only 16 out of the 324 available Caucasian MIBC cases with miRNA expression level quantification exhibited results from the tumour tissue ("Primary Tumour") and the corresponding non-tumour tissue ("Solid Tissue Normal"). Seven of the miRNAs found in the Discovery phase for MIBC were also found to be differentially expressed in a significant manner and in the same direction in this subset of TCGA data (Table 4).

**Table 4**

| **ID miRNA** | **ID miRNA (TCGA)** | **Mean read counts** | **log2 Fold Change** | **adj P (Bonferroni)** | **adj P (FDR)** |
|---|---|---|---|---|---|
| miR-30a-5p (SEQ ID no. 3) | miR-30a (SEQ ID no. 3) | 179888 | -2.496 | 5.28045E-21 | 1.26467E-19 |
| amiR-21-5p (SEQ ID no. 1) | miR-21 | 854585 | 1.717 | 9.28451E-09 | 1.9336E-08 |
| miR-106b-3p (SEQ ID no. 2) | miR-106b (SEQ ID no. 2) | 2614 | 1.091 | 9.0971E-06 | 7.14346E-06 |
| let-7c-5p (SEQ ID no. 4) | let-7c (SEQ ID no. 4) | 27205 | -1.896 | 0.0002 | 8.76786E-05 |
| miR-7-1-5p (SEQ ID no. 9) | miR-7-1 | 93 | 0.968 | 0.0005 | 0.0002 |
| miR-205-5p (SEQ ID no. 6) | miR-205 (SEQ ID no. 6) | 25289 | 1.956 | 0.0049 | 0.0015 |
| miR-25-3p (SEQ ID no. 8) | miR-25 (SEQ ID no. 8) | 47417 | 0.656 | 0.0801 | 0.0165 |
| miR-486-5p (SEQ ID no. 5) # | miR-486-2 # | 475 | -0.645 | 1.8177 | 0.2466 |
| miR-486-5p (SEQ ID no. 5) # | miR-486-1 # | 473 | -0.593 | 2.3635 | 0.3102 |
| miR-451a (SEQ ID no. 7) | miR-451a (SEQ ID no. 7) | 3849 | -0.221 | 6.7117 | 0.7409 |
| miR-146a-5p (SEQ ID no. 10) | miR-146a (SEQ ID no. 10) | 482 | -0.058 | 8.8212 | 0.9087 |

| | | | | | |
|---|---|---|---|---|---|
| # miRNA for which the locus could not be distinguished | | | | | |

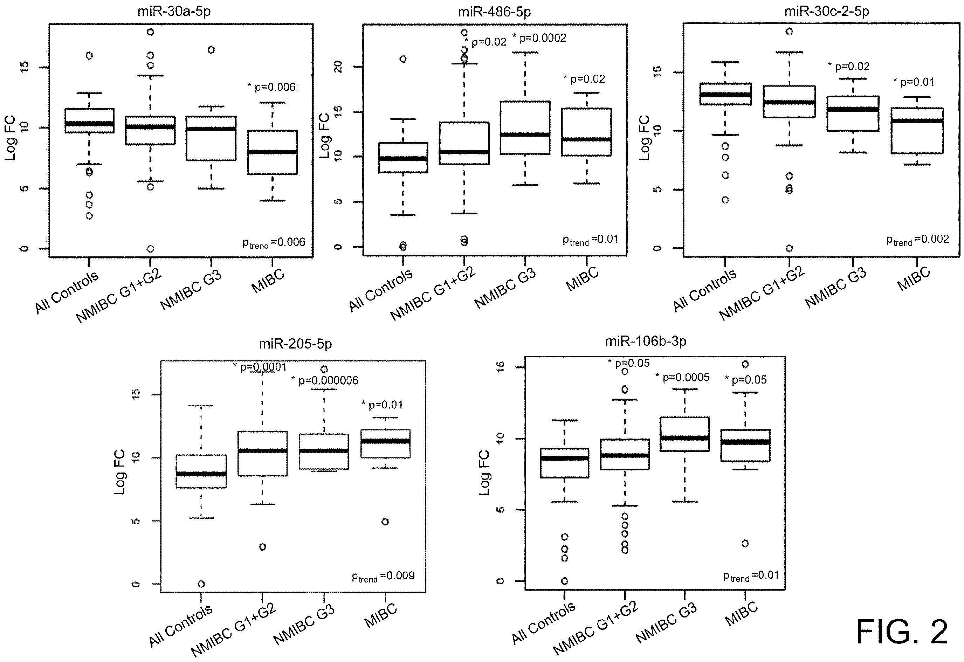

A significant increasing/decreasing trend (adjusted p <0.05) in expression levels from healthy controls to MIBC patients (p=0.006 and 0.01, respectively) was observed for two of the three miRNAs, miR-30a-5p (SEQ ID no. 3) and miR-486-5p (SEQ ID no. 5) significantly altered in the three BC subtypes. Significant trends were also observed for some miRNAs in common between NMIBC G1+G2 and G3: miR-30c-2-5p (SEQ ID no. 11); p=0.002, NMIBC G1+G2 and MIBC: miR-205-5p (SEQ ID no. 6); p=0.009, and NMIBC G3 and MIBC: miR-106b-3p (SEQ ID no. 2); p=0.01) (Table 5; Figure 2). The same analyses were also carried out considering the BC cases alone, confirming a significant decreasing trend for miR-10b-5p (SEQ ID no. 15), miR-98-5p (SEQ ID no. 22), miR-148b-3p (SEQ ID no. 16), miR-30a-5p (SEQ ID no. 3) and miR-30c-2-5p (SEQ ID no. 11) (p from 0.002 to 0.04).

**Table 5**

| **TargetID** | **Beta** | **P-value** | **Adj p-value (FDR)** |
|---|---|---|---|
| miR-10b-5p (SEQ ID no. 15) | -1.92 | 9.50E-05 | 0.002 |
| miR-224-5p (SEQ ID no. 20) | 1.95 | 7.54E-04 | 0.008 |
| miR-30c-2-5p (SEQ ID no. 11) | -1.72 | 2.03E-03 | 0.010 |
| miR-25-3p (SEQ ID no. 8) | 1.87 | 2.12E-03 | 0.010 |
| miR-7-1-5p (SEQ ID no. 9) | 2.06 | 2.48E-03 | 0.010 |
| miR-451a (SEQ ID no. 7) | 2.83 | 2.98E-03 | 0.010 |
| miR-30a-5p (SEQ ID no. 3) | -1.56 | 6.21E-03 | 0.018 |
| miR-205-5p (SEQ ID no. 6) | 1.47 | 8.85E-03 | 0.022 |
| miR-486-5p (SEQ ID no. 5) | 2.39 | 1.06E-02 | 0.024 |
| miR-106b-3p (SEQ ID no. 2) | 1.32 | 1.29E-02 | 0.026 |
| miR-98-5p (SEQ ID no. 22) | -0.98 | 1.97E-02 | 0.036 |
| let-7i-5p (SEQ ID no. 13) | 0.98 | 3.04E-02 | 0.051 |
| miR-148b-3p (SEQ ID no. 16) | -0.63 | 1.33E-01 | 0.2 0 5 |
| miR-146a-5p (SEQ ID no. 10) | 0.89 | 1.92E-01 | 0.264 |
| miR-185-5p (SEQ ID no. 18) | 0.54 | 2.02E-01 | 0.264 |
| miR-21-5p (SEQ ID no. 1) | 0.65 | 2.11E-01 | 0.264 |
| miR-183-5p (SEQ ID no. 17) | 0.56 | 2.37E-01 | 0.279 |
| let-7c-5p (SEQ ID no. 4) | -0.59 | 3.18E-01 | 0.354 |
| miR-151a-3p (SEQ ID no. 12) | 0.14 | 7.59E-01 | 0.799 |
| miR-200c-3p (SEQ ID no. 19) | 0.08 | 8.51E-01 | 0.851 |

Two models were then designed:
- model 0 including the classic BC risk factors (age and smoking habit);
- model 1 = model 0 plus miR-30a-5p (SEQ ID no. 3), let-7c-5p (SEQ ID no. 4) and miR-486-5p (SEQ ID no. 5), which characterise the three BC subtypes.

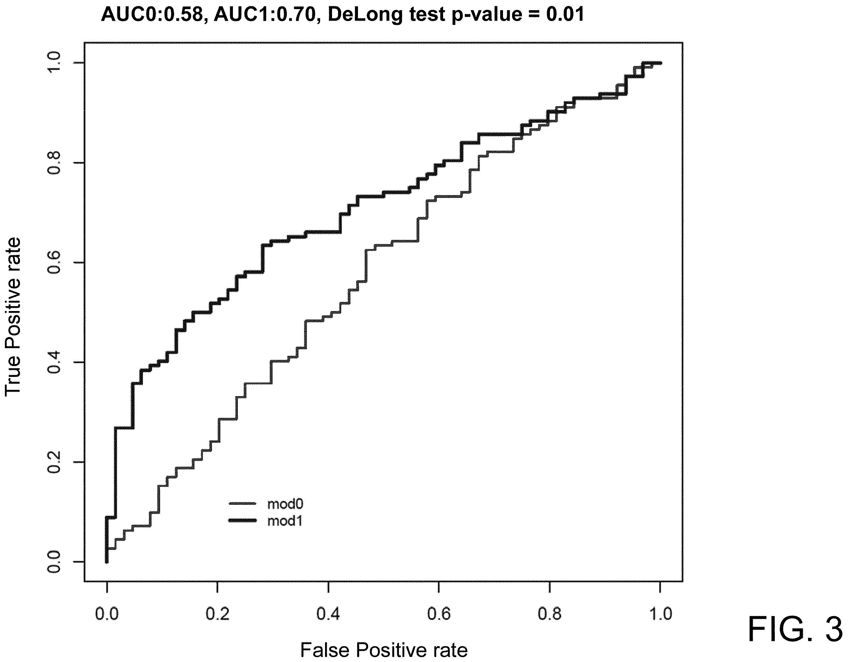

Model 1 showed a statistically significant improvement in discrimination between cases and controls compared to model 0 (AUC model 0 = 0.57, 95% CI: 0.49-0.67; AUC model 1 = 0.70, 95% CI: 0.63-0.78, DeLong's test p = 0.01, as shown in Figure 3). Considering only the 3 BC subgroups separately and including in each Model 1 only significant DEmiRNAs from Table 3, a statistically significant improvement in discrimination was observed for each subset of cases and controls (for NMIBC G1+G2 cases, AUC model 0 = 0.62, AUC model 1 = 0.73; DeLong's test p=0.02; for NMIBC G3 cases, AUC model 0 = 0.57, AUC model 1 = 0.95; DeLong's test p=1.15 × 10-6, for MIBC cases, AUC model 0 = 0.64, AUC model 1 = 0.99, DeLong's test p = 2.27 × 10-5) (data not shown).

Each of the selected miRNAs shows a large number of validated target genes (ranging from 66 to 913) significantly involved in several important KEGG pathways. Among these, for let-7c-5p (SEQ ID no. 4), miR-10b-5p (SEQ ID no. 15), and miR-200c -3p (SEQ ID no. 19), MAPK pathway is pointed out; for let-7c-5p (SEQ ID no. 4), and miR-106b-3p (SEQ ID no. 2), ErbB signalling pathway and cancer/bladder cancer pathways are pointed out. In particular, target genes of miR-30a-5p (SEQ ID no. 3), miR-486-5p (SEQ ID no. 5), miR-30c-2-5p (SEQ ID no. 11), miR-205-5p (SEQ ID no. 6) and miR-106b-3p (SEQ ID no. 2), whose expression levels showed a trend to over-representation from healthy controls to MIBC patients, were involved in several KEGG pathways, such as "Pathways in cancer_Homo sapiens" (hsa05200, adjusted p = 1.05 × 10-7), "MicroRNAs in cancer_Homo sapiens" (hsa05206; adjusted p = 2.97 × 10-7) and "Bladder cancer_Homo sapiens "(hsa05219; adjusted p = 3.38 × 10-5).

### References

[1] Turinetto V et al. H2AX phosphorylation level in peripheral blood mononuclear cells as an event-free survival predictor for bladder cancer. Molecular carcinogenesis. 2015. 55(11):1833-1842.
[2] Sacerdote C et al. Polymorphisms in the XRCC1 gene modify survival of bladder cancer patients treated with chemotherapy. Int. J. Cancer 2013; 133(8): 2004-2009.
[3] Critelli R et al. Detection of multiple mutations in urinary exfoliated cells from male bladder cancer patients at diagnosis and during follow-up. Oncotarget. 2016; 7(41): 67435-67448.
[4] Cordero F et al. Optimizing a massive parallel sequencing workflow for quantitative miRNA expression analysis. PLoS One. 2012; 7(2): e31630.
[5] Martina F et al. Peculiar Genes Selection: A new features selection method to improve classification performances in imbalanced data sets. PLoS One. 2017; 12(8): e0177475.
[6] Eisenberg E and Levanon EY. Human housekeeping genes, revisited. Trends in genetics. 2013; 29(10): 569-574.
[7] Dweep H et al. miRWalk--database: prediction of possible miRNA binding sites by "walking" the genes of three genomes. Journal of biomedical informatics. 2011; 44(5): 839-847.
[8] Chen EY et al. Enrichr: interactive and collaborative HTML5 gene list enrichment analysis tool. BMC Bioinformatics. 2013; 14:128.
[9] Love MI et al. Moderated estimation of fold change and dispersion for RNA-seq data with DESeq2. Genome biology. 2014; 15(12): 550.
[10] Liao JG and Chin KV. Logistic regression for disease classification using microarray data: model selection in a large p and small n case. Bioinformatics. 2007; 23(15): 1945-1951.
[11] Kuleshov MV et al. Enrichr: a comprehensive gene set enrichment analysis web server 2016 update. Nucleic acids research. 2016. 44(W1):W90-7.
[12] The Cancer Genome Atlas Research Network Comprehensive molecular characterization of urothelial bladder carcinoma. Nature. 2014; 507(7492):315-322.

SEQUENCE LISTING

<110>  Università degli Studi di Torino, Italian Institute for
       Genomic Medicine (IIGM)

<120>  UromiRNA per la diagnosi in vitro di tumore alla vescica

<130>  E6156-EC

<160>  23

<170>  PatentIn version 3.5

<210>  1
<211>  22
<212>  RNA
<213>  Homo sapiens

<400>   SEQ ID no. 1
uagcuuauca gacugauguu ga                                              22

<210>  2
<211>  22
<212>  RNA
<213>  Homo sapiens

<400>   SEQ ID no. 2
ccgcacugug gguacuugcu gc                                              22

<210>  3
<211>  22
<212>  RNA
<213>  Homo sapiens

<400>  SEQ ID no. 3
uguaaacauc cucgacugga ag                                              22

<210>  4
<211>  22
<212>  RNA
<213>  Homo sapiens

<400>  SEQ ID no. 4
ugagguagua gguuguaugg uu                                              22

<210>  5
<211>  22
<212>  RNA
<213>  Homo sapiens

<400>  SEQ ID no. 5
uccuguacug agcugccccg ag                                              22

<210>  6
<211>  22
<212>  RNA
<213>  Homo sapiens

<400>  SEQ ID no. 6
uccuucauuc caccggaguc ug                                              22


<210>  7
<211>  22
<212>  RNA
<213>  Homo sapiens

<400>  SEQ ID no. 7
aaaccguuac cauuacugag uu                                              22


<210>  8
<211>  22
<212>  RNA
<213>  Homo sapiens

<400>  SEQ ID no. 8
cauugcacuu gucucggucu ga                                              22


<210>  9
<211>  23
<212>  RNA
<213>  Homo sapiens

<400>  SEQ ID no. 9
uggaagacua gugauuuugu ugu                                             23


<210>  10
<211>  22
<212>  RNA
<213>  Homo sapiens

<400>  SEQ ID no. 10
ugagaacuga auuccauggg uu                                              22


<210>  11
<211>  23
<212>  RNA
<213>  Homo sapiens

<400>  SEQ ID no. 11
uguaaacauc cuacacucuc agc                                             23


<210>  12
<211>  21
<212>  RNA
<213>  Homo sapiens

<400>  SEQ ID no. 12
cuagacugaa gcuccuugag g                                               21


<210>  13
<211>  22
<212>  RNA
<213>  Homo sapiens

<400>  SEQ ID no. 13
ugagguagua guuugugcug uu                                                22

<210>  14
<211>  23
<212>  RNA
<213>  Homo sapiens

<400>  SEQ ID no. 15
uacccuguag aaccgaauuu gug                                               23

<210>  15
<211>  22
<212>  RNA
<213>  Homo sapiens

<400>  SEQ ID no. 16
ucagugcauc acagaacuuu gu                                                22

<210>  16
<211>  22
<212>  RNA
<213>  Homo sapiens

<400>  SEQ ID no. 17
uauggcacug guagaauuca cu                                                22

<210>  17
<211>  22
<212>  RNA
<213>  Homo sapiens

<400>  SEQ ID no. 18
uggagagaaa ggcaguuccu ga                                                22

<210>  18
<211>  23
<212>  RNA
<213>  Homo sapiens

<400>  SEQ ID no. 19
uaauacugcc ggguaaugau gga                                               23

<210>  19
<211>  21
<212>  RNA
<213>  Homo sapiens

<400>  SEQ ID no. 20
caagucacua gugguuccgu u                                                 21

<210>  20
<211>  20
<212>  RNA
<213>  Homo sapiens

<400>  SEQ ID no. 21
ggcuccuugg ucuaggggua                                                    20

<210>  21
<211>  22
<212>  RNA
<213>  Homo sapiens

<400>  SEQ ID no. 22
ugagguagua aguuguauug uu                                                 22

<210>  22
<211>  22
<212>  RNA
<213>  Homo sapiens

<400>  SEQ ID no. 23
cacuagauug ugagcuccug ga                                                 22

<210>  23
<211>  23
<212>  RNA
<213>  Homo sapiens

<400>  SEQ ID no. 24
ucccccaggu gugauucuga uuu                                                23

## Claims

1. An *in vitro* method for the diagnosis of bladder cancer, wherein the method comprises the following steps:
a) determining the expression levels of a series of miRNAs comprising at least miR-30a-5p (SEQ ID no. 3), miR-486-5p (SEQ ID no. 5), let-7c-5p (SEQ ID no. 4) in a sample of biological fluid from a subject;
b) comparing said expression levels with the expression levels of the same miRNAs in a reference sample, wherein a difference is indicative of bladder cancer.

2. The method according to claim 1, wherein said bladder cancer is muscle-invasive and said series of miRNAs further comprises: miR-205-5p (SEQ ID no. 6), miR-451 (SEQ ID no. 7), miR-25-3p (SEQ ID no. 8), miR-7-1-5p (SEQ ID no. 9) and said difference in expression is indicative of muscle-invasive bladder cancer.

3. The method according to claim 2, wherein said series of miRNAs further comprises: miR-21-5p (SEQ ID no. 1), miR-106b-3p (SEQ ID no. 2), miR-146a-5p (SEQ ID no. 10).

4. The method according to claim 2 or 3, wherein said expression levels of said miRNAs vary as follows with respect to a reference sample: miR-21-5p (SEQ ID no. 1) is increased, miR-106b-3p (SEQ ID no. 2) is increased, miR-30a-5p (SEQ ID no. 3) is decreased, let-7c-5p (SEQ ID no. 4) is decreased, miR-486-5p (SEQ ID no. 5) is increased, miR-205-5p (SEQ ID no. 6) is increased, miR-451a (SEQ ID no. 7) is increased, miR-25-3p (SEQ ID no. 8) is increased, miR-7-1-5p (SEQ ID no. 9) is increased, miR-146a-5p (SEQ ID no. 10) is increased.

5. The method according to claim 1, wherein said bladder cancer is Grade 1-2 non muscle-invasive and said series of miRNAs further comprises: miR-205-5p (SEQ ID no. 6), miR-30c-2-5p (SEQ ID no. 11), let-7i-5p (SEQ ID no. 13) and said difference in expression is indicative of Grade 1-2 non muscle-invasive bladder cancer.

6. The method according to claim 5, wherein said expression levels of said miRNAs vary as follows with respect to a reference sample: miR-30a-5p (SEQ ID no. 3) is decreased, let-7c-5p (SEQ ID no. 4) is increased, miR-486-5p (SEQ ID no. 5) is increased, miR-205-5p (SEQ ID no. 6) is increased, let-7i-5p (SEQ ID no. 13) is increased, miR-30c-2-5p (SEQ ID no. 11) is decreased.

7. The method according to claim 1, wherein said bladder cancer is Grade 3 non muscle-invasive and said series of miRNAs further comprises: miR-21-5p (SEQ ID no. 1), miR-106b-3p (SEQ ID no. 2), miR-30c-2-5p (SEQ ID no. 11), miR-151a-3p (SEQ ID no. 12), miR-200c-3p (SEQ ID no. 19), miR-183-5p (SEQ ID no. 17), miR-185-5p (SEQ ID no. 18), miR-10b-5p (SEQ ID no. 15), miR-224-5p (SEQ ID no. 20) and said difference in expression is indicative of Grade 3 non muscle-invasive bladder cancer.

8. The method according to claim 7, wherein said series of miRNAs further comprises: miR-98-5p (SEQ ID no. 22) miR-148b-3p (SEQ ID no. 16).

9. The method according to claim 7 or 8, wherein said expression levels of said miRNAs vary as follows with respect to a reference sample: miR-21-5p (SEQ ID no. 1) is increased, miR-106b-3p (SEQ ID no. 2) is increased, miR-486-5p (SEQ ID no. 5) is increased, miR-30c-2-5p (SEQ ID no. 11) is decreased, miR-151a-3p (SEQ ID no. 12) is increased, miR-200c-3p (SEQ ID no. 19) is increased, miR-183-5p (SEQ ID no. 17) is increased, miR-185-5p (SEQ ID no. 18) is increased, miR-10b-5p (SEQ ID no. 15) is decreased, miR-224-5p (SEQ ID no. 20) is increased, miR-30a-5p (SEQ ID no. 3) is decreased, let-7c-5p (SEQ ID no. 4) is increased, miR-98-5p (SEQ ID no. 22) is increased.

10. The method according to any of claims 1 to 9, wherein said biological fluid is urine.
